(19) Europäisches Patentamt
European Patent Office
Office européen des brevets

(11) Veröffentlichungsnummer: **0 302 331 B1**

(12) **EUROPÄISCHE PATENTSCHRIFT**

(45) Veröffentlichungstag der Patentschrift: **16.09.92**

(51) Int. Cl.$^5$: **C07C 67/14**, C07C 69/767, C07C 69/92, C07C 321/28

(21) Anmeldenummer: **88111895.4**

(22) Anmeldetag: **23.07.88**

(54) **Ester von Arylbisperfluoralkylcarbinolen, Verfahren zur Herstellung dieser Verbindungen und der zugrundeliegenden Arylbisperfluoralkylcarbinole.**

(30) Priorität: **05.08.87 DE 3725941**

(43) Veröffentlichungstag der Anmeldung:
**08.02.89 Patentblatt 89/06**

(45) Bekanntmachung des Hinweises auf die Patenterteilung:
**16.09.92 Patentblatt 92/38**

(84) Benannte Vertragsstaaten:
**CH DE FR GB IT LI NL**

(56) Entgegenhaltungen:
**EP-A- 0 074 057**
**EP-A- 0 301 444**
**US-A- 3 293 286**

**CHEMICAL ABSTRACTS, Band 82, Nr. 13, 31. März 1975, Seite 520, Spalte 1, Zusammenfassung Nr. 86321h, Columbus, Ohio, USA; J. G. MOORE et al: "Reactions of sodium perfluorotertiary alkoxides with reactive halides"**

(73) Patentinhaber: **HOECHST AKTIENGESELLSCHAFT**
**Postfach 80 03 20**
**W-6230 Frankfurt am Main 80(DE)**

(72) Erfinder: **Kruse, Alfred, Dr.**
**Hornauer Strasse 199**
**W-6233 Kelkheim (Taunus)(DE)**
Erfinder: **Siegemund, Günter, Dr.**
**Frankfurter Strasse 21**
**W-6238 Hofheim am Taunus(DE)**
Erfinder: **Ruppert, Ingo, Dr.**

**verstorben(DE)**

**EP 0 302 331 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF THE AMERICAN CHEMICAL SOCIETY, Band 97, Nr. 21, October 1975, Seite 6137-6144, Washington, DC, USA; J. C. MARTIN et al: "The Reactions of Secondary Amides with a Diaryldialkoxysulfurane. A Selective Method of the Rapid Cleavage of Secondary Amides"

CHEMICAL ABSTRACTS, Band 95, Nr. 9, 31. August 1981, Seite 734, Spalte 2, Zusammenfassung Nr. 80415p, Columbus, Ohio, USA; N. V. KONSTANTINOVA et al: "Acylation of bis(trifluoromethyl)phenylcarbinols substituted in the ring"

JOURNAL OF THE AMERICAN CHEMICAL SOCIETY, Band 93, Nr. 9, 5. Mai 1971, Seiten 2339-3341, Washington, DC, USA; J. C. MARTIN et al: "Sulfurances. I. A Stable Tetracoordinate Tetracovalent Sulfur Compound in Solution"

## Beschreibung

Die Erfindung betrifft Ester von Arylbisperfluoralkylcarbinolen sowie ein Verfahren zu deren Herstellung und der Herstellung der zugrundeliegenden Arylbisperfluoralkylcarbinole, also von tertiären, Fluor enthaltenden Alkoholen. Als Ausgangsmaterialien werden Arylcarbonsäurehalogenide oder auch Arylperfluoralkylketone eingesetzt, die durch Reaktion mit Perfluoralkylhalogeniden in Gegenwart von Phosphorigsäuretriamiden die Arylcarbonsäureester der Arylbisperfluoralkylcarbinole oder je nach Versuchsführung die Carbinole selbst ergeben.

Arylbisperfluoralkylcarbinole sind wichtige Zwischenprodukte für die Herstellung von Kunststoffen, Inertflüssigkeiten, Arzneimitteln und Pflanzenschutzmitteln.

Arylbisperfluoralkylcarbinole der allgemeinen Formel I lassen sich durch Funktionalisierung der entsprechenden Bisperfluoralkylketone II oder alternativ aus Arylperfluoralkylketonen III erzeugen (Formeln s. Formelblatt). In den beiden Gruppen der Formel II kann n gleich oder verschieden sein und bedeutet 0 oder eine ganze Zahl.

Die günstigste Herstellungsmethode für Verbindungen mit n = 0 ist die Umsetzung des aromatischen Ringsystems mit Hexafluoraceton II (n = 0) unter der Katalyse einer Lewissäure nach Friedel-Crafts (J. Org. Chem. 30, 998-1007 (1965)). Die Position, in der die Hexafluorisopropanolgruppe an den aromatischen Ring gebunden wird, ist von der Natur und Stellung vorhandener Substituenten abhängig, so daß sich mit diesem Verfahren nur bestimmte Substitutionstypen der fluorhaltigen Alkohole der Formel I erhalten lassen. Weitere Nachteile dieses Verfahrens sind die mögliche Bildung von Stellungsisomeren bei der Substitution am Arylring und dadurch verursachte Reinigungsprobleme, eine mögliche mehrfache Substitution sowie die Tatsache, daß die entstandenen Alkohole in einigen Fällen unter den Reaktionsbedingungen in unerwünschter Weise weiterkondensieren. Die Anwendung dieses Verfahrens auf die homologen Ketone des Hexafluoracetons zur Herstellung von Alkoholen der allgemeinen Formel I, in denen n mindestens 1 bedeutet, liefert deutlich schlechtere Ausbeuten (s. DE-PS 1 210 775).

Will man den Perfluoralkylcarbinolrest an einer ganz bestimmten Stelle des aromatischen Rings einführen, muß man zunächst eine entsprechende Arylorganometallverbindung synthetisieren und diese in einem zweiten Schritt an das perfluorierte Keton addieren (s. DE-PS 1 210 775).

Nachteilig und limitierend für beide Verfahren ist die schlechte Verfügbarkeit der Ausgangsstoffe, namentlich der Perfluoralkylketone, und deren Toxizität.

Es ist weiter bekannt, daß sich derartige Alkohole auch durch Einführung eines Perfluoralkylrestes in ein Arylperfluoralkylketon III mittels einer entsprechenden Perfluoralkylorganometallverbindung herstellen lassen (Tetrahedron Lett. 26, 5243-46 (1985)). Nachteilig ist hier die aufwendige Herstellung und Labilität der zunächst herzustellenden Perfluoralkylmetallverbindung sowie die schlechte Reproduzierbarkeit der publizierten Ergebnisse (Tetrahedron Lett. 26, 5245, (Fußnote 4).

In der Literaturstelle Chemical Abstracts 82 (1975) 86321 H (G.J. Moore et al., J. Fluorine Chem. 5 - (1975) 77-81) ist die Verbindung (Pentafluor-benzoesäure-(2,2,2-trifluor-1-pentafluorphenyl-1-trifluormethyl-äthyl)-ester beschrieben. Die Verbindungen 1,1,1,3,3,3,-Hexaflour-2-phenyl-2-propyl -2-benzoat und 1,1,1,3,3,3-Hexaflour-2-phenyl-propyl-2-(4-chlorbenzoat) sind aus J. Am. Chem. 97 (1975) 6138-6144 bekannt.

Gegenstand der Erfindung sind nun Ester von Arylbisperfluoralkylcarbinolen der allgemeinen Formel IV (s. Formelblatt), worin die Reste $R^1$ bis $R^5$ gleich oder verschieden sind und Wasserstoff, Alkyl mit 1 bis 6 Kohlenstoffatomen, das perfluoriert sein kann, Halogen, Alkoxy oder Alkylthio mit jeweils 1 bis 6 Kohlenstoffatomen sind, und n in beiden Fällen gleich oder verschieden ist und 0 oder eine ganze Zahl von 1 bis 5 bedeutet, sowie ein Verfahren zu deren Herstellung und der zugrundeliegenden Carbinole, das dadurch gekennzeichnet ist, daß man Carbonylverbindungen der allgemeinen Formel Va (s. Formelblatt), in der X Fluor, Chlor, Brom oder eine Perfluoralkylgruppe $(CF_2)_nCF_3$ ist, mit Perfluoralkylhalogeniden der allgemeinen Formel $CF_3$-$(CF_2)_n$-Y (VI), worin Y Chlor, Brom oder Jod ist und wobei n jeweils die angegebene Bedeutung hat, in Gegenwart von einer - bezogen auf die Verbindung Va - mindestens äquimolaren Menge an Phosphorigsäuretrisdialkylamiden (in anderen Worten Tris(dialkylamino)phosphanen) der allgemeinen Formel $P(N(Alkyl)_2)_3$ (VII) umsetzt und dabei die gewünschten Verbindungen der Formel VIII (s. Formelblatt) erhält, in der $R^1$ bis $R^5$ und n die angegebene Bedeutung haben und Z Wasserstoff oder den Arylcarbonsäurerest der Formel V (s. Formelblatt) bedeutet. Von den Verbindungen der Formel IV sind diejenigen ausgenommen, bei denen alle $R^1$ bis $R^5$ eine andere Bedeutung als jeweils Wasserstoffatome oder Fluoratome haben, wenn n = 0 ist, sowie 1,1,1,3,3,3-Hexafluor-2-phenyl-propyl-2-(4-chlorbenzoat).

Unter geeigneten Reaktionsbedingungen werden bei der erfindungsgemäßen Reaktion auf die Arylcarbonsäurehalogenide sukzessive zwei Perfluoralkylgruppen oder auf ein Arylperfluoralkylketon, das auch bei der Reaktion mit dem Arylcarbonsäurehalogenid als Zwischenprodukt gebildet wird, eine Perfluoralkylgruppe übertragen. Nach dieser Ausführungsform lassen sich auch fluorierte Alkohole mit zwei verschiedenen Perfluoralkylgruppen herstellen.

Die Carbonsäurehalogenide, von denen die Carbonsäurechloride und -fluoride bevorzugt sind, und die Arylperfluoralkylketone können unsubstituiert sein oder einen oder mehrere gleiche oder unterschiedliche Substituenten $R^1$ bis $R^5$ mit anderer Bedeutung als Wasserstoff tragen. Geeignete Substituenten sind z.B. Alkyl-, Alkoxy- und Alkylthioreste jeweils mit 1 bis 6, insbesondere 1 bis 3 Kohlenstoffatomen, wobei die Alkylreste perfluoriert sein können, sowie Halogen (Fluor, Chlor, Brom, Jod).

Zweckmäßig sind an den aromatischen Ring nicht mehr als drei und vorzugsweise höchstens zwei Substituenten $R^1$ bis $R^5$ mit anderer Bedeutung als Wasserstoff gebunden. Die Alkyl-, Alkoxy- und Alkylthiosubstituenten enthalten zweckmäßig zusammen höchstens 4 C-Atome und können geradkettig oder verzweigt sein.

Als Perfluoralkylhalogenide werden im allgemeinen Verbindungen mit 1 bis 6, insbesondere 1 bis 3 C-Atomen verwendet, vorzugsweise $CF_3Br$ und die homologen Perfluoralkyljodide der allgemeinen Formel $C_mF_{2m+1}J$ mit m = 1 bis 6 bzw. bis 3.

Als Phosphorigsäuretrisdialkylamide (VII) kommen vor allem die niederen Alkylverbindungen in Frage, insbesondere solche mit $C_1$-$C_4$-Alkyl, wie Trisdimethylaminophosphan, Trisdiäthylaminophosphan und Trisdipropyl- bzw. -isopropylaminophosphan; bevorzugt wird Trisdiäthylaminophosphan $P(N(CH_2CH_3)_2)_3$ verwendet. Dieses läßt sich sehr einfach in hohen Ausbeuten durch Reaktion von Phosphortrichlorid mit Diäthylamin in einem gegenüber den Reaktionsteilnehmern inerten Lösungsmittel, z.B. einem aliphatischen, cycloaliphatischen oder aromatischen Kohlenwasserstoff oder einem Kohlenwasserstoffgemisch, erzeugen. Die Dialkylaminogruppen können gleiche oder verschiedene Alkylgruppen enthalten.

Bei der Umsetzung des Arylcarbonsäurehalogenids mit einem Perfluoralkylhalogenid wird unter dem Einfluß des Phosphorigsäuretrisdialkylamids formal ein Mol Halogen bzw. gemischtes Halogen abgespalten und ein salzartiges Addukt aus dem Phosphorigsäuretriamid und Halogen gebildet. Das Carbonsäurehalogenid wird auf diese Weise zunächst in ein Arylperfluoralkylketon übergeführt. Diese Reaktion ist bereits in der älteren DE-Patentanmeldung 88 111 896.2, HOE 87/F 221 vom 29.7.1987, beschrieben. Dieses Keton reagiert in Anwesenheit eines Überschusses an Phosphorigsäuretriamid und Perfluoralkylhalogenid unter Addition einer zweiten Perfluoralkylgruppe und Bildung des Anions des entsprechenden tertiären Alkohols weiter.

Wird das Carbonsäurehalogenid also mit der doppelt-molaren Menge an Perfluoralkylhalogenid und Phosphorigsäuretriamid zur Reaktion gebracht, lassen sich die tertiären Alkohole aus dem zunächst entstandenen Alkoholat der Formel IX (s. Formelblatt) nach Zugabe einer Säure wie Fluor-, Chlor-oder Bromwasserstoffgas direkt erhalten. Diese Gase können gegebenenfalls auch in Alkoholen oder Wasser gelöst ein, wobei beim Arbeiten mit wäßrigen Systemen zweckmäßig oberhalb der Temperatur der Bildung von Eiskristallen gearbeitet wird. Dieses Verfahren ergibt jedoch in einigen Fällen unbefriedigende Ausbeuten, so daß es zuweilen vorteilhafter ist, den tertiären Alkohol aus dem entsprechenden Ester durch alkalische Hydrolyse herzustellen.

Die Veresterung der zunächst gebildeten Alkoholate mit dem Carbonsäurehalogenid verläuft sehr glatt, und bei geeigneter Reaktionsführung werden die entsprechenden Ester in hohen Ausbeuten erhalten. Durch die Veresterung der tertiären Alkoholate läßt sich die Hydrolyse der Reaktionsmischung zur Freisetzung der Alkohole vermeiden und die Aufarbeitung vereinfachen. Des weiteren werden mögliche Sekundärreaktionen der Alkohole unterbunden, da die Ester unter den Bedingungen der Aufarbeitung chemisch inert sind.

Zur Einführung von zwei unterschiedlichen Perfluoralkylresten geht man zweckmäßig von Arylperfluoralkylketonen aus, setzt diese mit Perfluoralkylhalogenid und Phosphorigsäuretriamid um und überführt das entstandene Alkoholat IX durch Zugabe einer Säure, wie oben ausgeführt, in das Carbinol oder durch weitere Umsetzung mit einem Arylcarbonsäurehalogenid Va in den Ester der Formel IV.

Die Umsetzung der Arylcarbonsäurehalogenide oder Arylperfluoralkylketone mit Perfluoralkylhalogenid in Gegenwart von Phosphorigsäuretriamid wird im allgemeinen bei Temperaturen von etwa -100 °C bis +40 °C durchgeführt. Die kurzkettigen Perfluoralkylhalogenide reagieren meist schon sehr rasch bei -78 °C. Bei den Perfluoralkylhalogeniden mit mindestens 2 C-Atomen ist es oft notwendig, die Reaktionstemperaturen zu erhöhen, um eine zügige Umsetzung zu erreichen; bevorzugt werden dann Temperaturen oberhalb -40 °C und z.B. bis +20 °C. Die Reaktionsdauer ist bekanntlich von den übrigen Bedingungen, insbesondere der Temperatur, abhängig. Die Reaktion ist im allgemeinen im Zeitraum von wenigen Minuten bis zu mehreren Stunden beendet.

Die Umsetzungen werden im allgemeinen ohne Anwendung von Überdruck ausgeführt. Jedoch kann es in einzelnen Fällen z.B. bei der Umsetzung der Perfluormethylhalogenide, zweckmäßig sein, auch bei erhöhtem Druck zu arbeiten, vor allem wenn oberhalb der Siedetemperatur (bei Normaldruck) des Perfluoralkylhalogenids gearbeitet wird. Praktisch wird man dann also bei mindestens dem Eigendruck

arbeiten.

Zweckmäßig wird das vorliegende Verfahren unter wasserfreien Bedingungen in Gegenwart eines gegenüber den Reaktionsteilnehmern inerten Lösungs- oder Verdünnungsmittels durchgeführt. Als solche kommen vor allem aprotische Flüssigkeiten zum Einsatz. Verwendet werden z.B. halogenierte Kohlenwasserstoffe wie Methylenchlorid, Tetrachloräthan, Nitrile, z.B. Acetonitril oder dessen Homologe oder Benzonitril, Ester wie Diäthylcarbonat oder Äthylencarbonat und Äther wie Tetrahydrofuran oder Dimethoxyäthan. Das Lösungsmittel sollte möglichst wasserfrei sein.

Es ist von Vorteil, während der gesamten Dauer der Reaktion, z.B. durch Rühren, für eine gute Durchmischung des Ansatzes zu sorgen sowie durch Wahl eines geeigneten Lösungsmittels die salzartigen Zwischen- und Begleitprodukte in Lösung zu halten.

Das erfindungsgemäße Verfahren kann beispielsweise so durchgeführt werden, daß man Lösungsmittel bzw. Verdünnungsmittel und zwei Komponenten vorlegt und die dritte zudosiert. Für die Herstellung der Ester aus dem Arylcarbonsäurehalogenid ist es günstig, Phosphorigsäuretriamid und Perfluoralkylhalogenid vorzulegen und das Carbonsäurehalogenid langsam zuzugeben. So ist ein ständiger Überschuß an Reagenzien, bezogen auf das Carbonsäurehalogenid, zu Beginn der Reaktion gewährleistet, damit das intermediär gebildete Keton sofort zum tertiären Alkohol weiter umgesetzt werden kann. Dieser reagiert dann unter den vorhandenen Reaktionsbedingungen mit weiterem Carbonsäurehalogenid zum Ester. Für die Herstellung der tertiären Alkohole aus den Arylperfluoralkylketonen ist der Modus und die Reihenfolge der Vereinigung der Komponenten beliebig. Essentiell ist jedoch in jedem Fall die anschließende Zugabe einer Protonensäure zum Reaktionsgemisch oder die Hydrolyse desselben, da andernfalls eine Weiterreaktion der Alkoholate mit dem Phosphoniumsalz eintreten kann.

Für die beiden geschilderten Reaktionen werden die Reagenzien in einer zur Carbonylverbindung mindestens stöchiometrischen Menge, oft in einem Überschuß bis zu 20 %, eingesetzt.

Wenn man bei der Herstellung der fluorierten tertiären Alkohole von Carbonsäurehalogeniden ausgeht, kann beispielsweise so verfahren werden, daß Lösungsmittel bzw. Verdünnungsmittel, Carbonsäurehalogenid und Perfluoralkylhalogenid vorgelegt und das Phosphorigsäuretriamid als letzte Komponente zugegeben wird. Wie aus der Stöchiometrie der Reaktion zu ersehen ist, werden die Reagenzien hier im Verhältnis zum Carbonsäurehalogenid nun mindestens in der doppelt molaren Menge eingesetzt, wobei ebenfalls ein Überschuß über die stöchiometrische Menge bis zu 20 % möglich ist.

Die Aufarbeitung des Reaktionsgemisches erfolgt vorteilhaft durch destillative Trennung der Komponenten. Die Aufarbeitung der Ester, in der Regel aber nicht der Alkohole, kann oft in einfacherer Weise und problemlos durch eine Extraktion erfolgen, indem man sie vom gleichzeitig entstandenen Phosphorigsäuretriamid-Halogen-Addukt (in anderen Worten ein Phosphoniumsalz) abtrennt. Bei der Zugabe eines unpolaren Lösemittels, z.B. eines Kohlenwasserstoffs wie Hexan, zum Reaktionsgemisch erhält man zwei Phasen, wobei sich in der oberen Phase die Perfluoralkylverbindung befindet und die untere Phase im wesentlichen das als Begleitprodukt anfallende, im Kohlenwasserstoff nicht lösliche Phosphoniumsalz enthält.

Die Aufarbeitung durch Extraktion ist für eine quantitative Isolierung der Alkohole nicht geeignet. Vielmehr empfiehlt es sich, das Reaktionsgemisch unter vermindertem Druck vom Lösungsmittel zu befreien und den Rückstand mit Wasser zu behandeln. Die Perfluoralkylverbindungen scheiden sich dann als wasserunlösliche Phase ab und können nach Abtrennung durch Destillation gereinigt werden. Dieses Verfahren ist auch für die Isolierung der Ester geeignet. Die Phosphoniumsalze lassen sich wegen ihrer guten Löslichkeit in organischen Lösungsmitteln anschließend leicht wieder aus der wäßrigen Phase extrahieren, z.B. mit einem Lösungsmittel wie $CH_2Cl_2$.

Die Strukturen der Verbindungen gemäß den Beispielen 1 bis 10 und deren physikalische Daten sind in der Tabelle zusammengefaßt.

**Beispiele**

1) In einem Rundkolben wurden unter Ausschluß von Feuchtigkeit bei ca. -70 °C zu einer Lösung von 31 g (0,25 mol) Benzoylfluorid in 150 ml $CH_2Cl_2$ 41 g (0,27 mol) Trifluormethylbromid einkondensiert. Dann wurden 62 g (0,25 mol) Phosphorigsäuretrisdiäthylamid zugegeben. Es wurde 4 Stunden bei ca. -70 °C gerührt und dann langsam erwärmt. Nach Zugabe der gleichen Volumenmenge Hexan zum Reaktionsgemisch bildeten sich 2 Phasen. Nach Phasentrennung wurde die untere Phase sorgfältig mit Hexan extrahiert. Die vereinigten Hexanphasen wurden eingeengt und unter vermindertem Druck destilliert. Es wurden 35,1 g (81 %) 1,1,1,3,3,3-Hexafluor-2-phenyl-propyl-2-benzoat erhalten. Umkristallisation aus iso-Propanol lieferte farblose Kristalle.

2) In einem Rundkolben wurden unter Ausschluß von Feuchtigkeit bei ca. -70 °C zu einer Lösung von 70

g (0,28 mol) Phosphorigsäuretrisdiäthylamid in 150 ml $CH_2Cl_2$ 45 g (0,3 mol) Trifluormethylbromid einkondensiert. Bei ca. -70 ° C wurden dann 39 g (0,25 mol) m-Tolylsäurechlorid in 50 ml $CH_2Cl_2$ innerhalb von 4 Stunden zugetropft. Es wurde noch 3 Stunden bei -70 ° C gerührt und dann erwärmt. Das Reaktionsgemisch wurde, wie im Beispiel 1 beschrieben, aufgearbeitet. Es wurden 39 g (83 %) 1,1,1,3,3,3-Hexafluor-2-(3-methylphenyl)-propyl-2-(3-methylbenzoat) erhalten. Umkristallisation aus iso-Propanol lieferte farblose Kristalle.

3) In einem Rundkolben wurden unter Ausschluß von Feuchtigkeit 74 g (0,3 mol) Phosphorigsäuretrisdiäthylamid in 150 ml $CH_2Cl_2$ vorgelegt. Bei ca. 0 ° C wurden 74 g (0,3 mol) Pentafluoräthyljodid zudosiert, dann 44 g (0,25 mol) o-Chlorbenzoylchlorid in 50 ml $CH_2Cl_2$ langsam zugetropft. Nach 8 Stunden wurde auf Raumtemperatur erwärmt. Das Reaktionsgemisch wurde unter vermindertem Druck vom Lösungsmittel befreit und der Rückstand in das dreifache Volumen Wasser eingebracht. Die organische Phase wurde abgetrennt, getrocknet und destilliert. Es wurden 49 g (76 %) 3-(2-Chlorphenyl)-1,1,1,2,2,4,4,5,5,5-decafluorpentyl-3-(2-chlorbenzoat) erhalten.

4) bis 10) Die Herstellung der Verbindungen gemäß diesen Beispielen, deren Struktur, physikalische Daten und Analysenwerte ergeben sich aus der Tabelle, wobei R den oder die angegebenen Reste $R^1$ bis $R^5$ der Formel IV darstellt.

11)

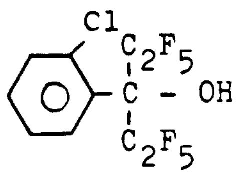

In einem Rundkolben wurden unter Ausschluß von Feuchtigkeit 74 g (0,3 mol) Phosphorigsäuretrisdiäthylamid in 150 ml $CH_2Cl_2$ vorgelegt. Bei ca. -20 ° C wurden 74 g (0,3 mol) Pentafluoräthyljodid und 26,4 g (0,15 mol) o-Chlorbenzoesäurechlorid zugegeben. Das Gemisch wurde auf 0 ° C erwärmt und etwa 8 Stunden bei dieser Temperatur gerührt. Dann wurden 10 g Chlorwasserstoffgas eingeleitet, und es wurde noch eine weitere Stunde bei 0 ° C gerührt. Das Reaktionsgemisch wurde unter vermindertem Druck vom Lösungsmittel befreit und in das dreifache Volumen Wasser eingebracht. Die organische Phase wurde abgetrennt, getrocknet und destilliert. Man erhielt 34,6 g (62 %) 3-(2-Chlorphenyl)-1,1,1,2,2,4,4,5,5,5-decafluorpentan-3-ol vom Kp. 66 ° C/2 mbar.

| Analyse: | | | | |
|---|---|---|---|---|
| Ber.<br>Gef. | C 34,89<br>C 35,1 | H 1,33<br>H 1,2 | Cl 9,36<br>Cl 9,7 | F 50,18<br>F 50,0 |

$^{19}F$-NMR($CDCl_3$): - 78,5 (m, 3F, $CF_3$), - 120,3 (m, 2F, $CF_2$)

12)

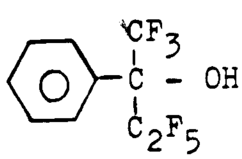

a) Es wurde zunächst das Ausgangsmaterial wie folgt hergestellt: In einem Rundkolben wurden unter Schutzgas bei ca. -20 ° C 35 g (0,25 mol) Benzoylchlorid in 150 ml $CH_2Cl_2$ vorgelegt. Es wurden zunächst 67 g (0,27 mol) Pentafluoräthyljodid einkondensiert und dann 66,7 g (0,27 mol) Phosphorigsäuretrisdiäthylamid zudosiert. Anschließend wurde das Reaktionsgemisch noch 5 Stunden bei 0 ° C gerührt. Nach Zugabe der gleichen Volumenmenge an Hexan zum Reaktionsgemisch bildeten sich 2 Phasen. Nach Phasentrennung wurde die untere Phase sorgfältig mit Hexan extrahiert; die vereinigten Hexanphasen wurden eingeengt und unter vermindertem Druck destilliert. Es wurden 20,8 g (58 %

Ausbeute) an Pentafluoräthylphenylketon vom Kp. 76-77 ° C/40 mbar erhalten.

b) In einem Rundkolben wurden unter Ausschluß von Feuchtigkeit 56 g (0,25 mol) Pentafluoräthylphenylketon in 150 ml $CH_2Cl_2$ vorgelegt. Bei -70 ° C wurden 46 g (0,3 mol) Trifluormethylbromid einkondensiert und 74 g (0,3 mol) Phosphorigsäuretrisdiäthylamid zugegeben. Es wurde 5 Stunden bei -70 ° C gerührt. Dann wurden 15 g (0,4 mol) Chlorwasserstoffgas eingeleitet und das Reaktionsgemisch langsam auf Raumtemperatur gebracht. Das Lösungsmittel wurde unter vermindertem Druck verdampft und der Rückstand in das dreifache Volumen Wasser eingetragen. Die organische Phase wurde abgetrennt und nach dem Trocknen destilliert. Man erhielt 41,1 g (56 %) 1,1,1,3,3,4,4,4-Octafluor-2-phenylbutan-2-ol vom Kp 66-68 ° C/20 mbar als farblose Flüssigkeit.

| Analyse: | | | |
|---|---|---|---|
| Ber.<br>Gef. | C 40,83<br>C 40,6 | H 2,06<br>H 2,1 | F 51,67<br>F 51,1 |

$^{19}$F-NMR($CDCl_3$): - 74.5 (m,3F,$CF_3$), -78.8 (m,3F,$\underline{CF_3}$-$CF_2$-), -121,3 (m,2F,$CF_2$)

13)

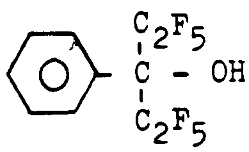

In einem Rundkolben wurde eine Lösung von 31,4 g (0,07 mol) des Esters des Beispiels 4 in 50 ml Tetrahydrofuran mit einer Lösung von 8 g (0,2 mol) Natriumhydroxid in 40 g Wasser versetzt. Das Gemisch wurde unter intensivem Rühren 90 Minuten unter Rückfluß erhitzt. Nach dem Abkühlen wurde angesäuert,die organische Phase abgetrennt und getrocknet. Die Destillation der organischen Phase lieferte17,1 g (71 %) 1,1,1,2,2,4,4,5,5,5-Decafluor-3-phenyl-pentan-3-ol vom Kp 78-79 ° C/13 mbar als farblose Flüssigkeit.

**Tabelle**

Beispiele 1-10

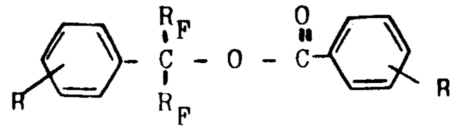

| Beisp. | R | $R_F$ | Verfahren analog Beispiel | Ausbeute | Siedepunkte °C/mbar Fp °C | C (ber.) gef. | H (ber.) gef. | F (ber.) gef. | $^{19}$F-NMR [ppm] $CF_3$ | $CF_2$ | IR [$cm^{-1}$] C = O |
|---|---|---|---|---|---|---|---|---|---|---|---|
| 1 | H | $CF_3$ | | 81 % | 96–97/0,1<br>Fp 48–49 | (55,18)<br>55,0 | (2,89)<br>2,9 | (32,74)<br>32,7 | -70,5 | - | 1760 |
| 2 | 3-$CH_3$ | $CF_3$ | | 83 % | 130–4/1<br>Fp 58–59 | (57,45)<br>57,2 | (3,75)<br>3,8 | (30,29)<br>30,5 | -70,5 | - | 1755 |
| 3 | 2-Cl | $C_2F_5$ | | 76 % | 132–4/0,1 | (41,81)<br>42,0 | (1,56)<br>1,4 | (36,74)<br>36,8 | -77,8 | -108,5 | 1770 |
| 4 | H | $C_2F_5$ | 3 | 51 % | 111-2/0,2 | (48,23)<br>48,7 | (2,25)<br>2,4 | (42,38)<br>42,5 | 77,6 | -110,1 | 1765 |
| 5 | 3-$CH_3$ | $C_2F_5$ | 3 | 47 % | 105–8/0,2 | (50,43)<br>50,7 | (2,96)<br>2,9 | (39,89)<br>40,2 | -77,6 | -110 | 1760 |
| 6 | 3,4($CH_3$)$_2$ | $CF_3$ | 2 | 88 % | 147–9/0,1<br>Fp 77–79 | (59,41)<br>59,9 | (4,49)<br>4,8 | (28,19)<br>28,5 | -70,6 | - | 1755 |
| 7 | 3-O-$CH_3$ | $CF_3$ | 2 | 63 % | 127–9/0,1 | (52,95)<br>53,5 | (3,46)<br>3,7 | (27,92)<br>27,5 | -70,4 | - | 1755 |
| 8 | 2-Cl | $CF_3$ | 2 | 87 % | 112–4/0,1<br>Fp 76 | (46,07)<br>46,2 | (1,93)<br>1,9 | (27,32)<br>27,4 | -69,3 | - | 1670 |
| 9 | 3-F | $CF_3$ | 2 | 48 % | 90–3/0,3 | (48,99)<br>48,8 | (2,06)<br>2,1 | (38,74)<br>38,4 | -70,6 | - | 1765 |
| 10 | 3-F | $C_2F_5$ | 3 | 55 % | 95–8/0,1 | (44,48)<br>44,4 | (1,66)<br>1,7 | (46,91)<br>47,4 | -77,5 | -110 | 1770 |

Formelblatt

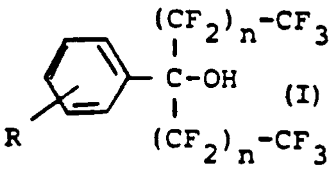
(I)

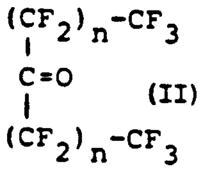
(II)

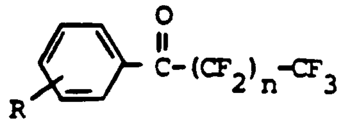
(III)

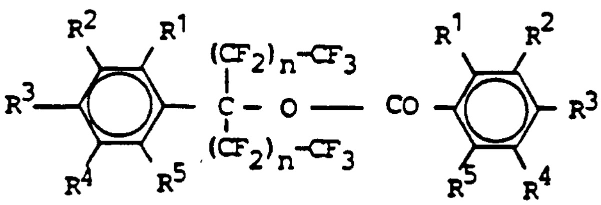
(IV)

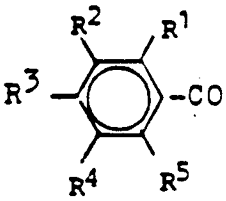
(V)

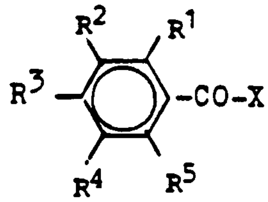
(Va)

$CF_3-(CF_2)_n-Y$ (VI)

$P(N(Alkyl)_2)_3$ (VII)

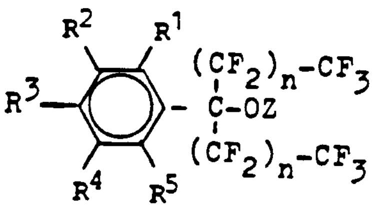
(VIII)

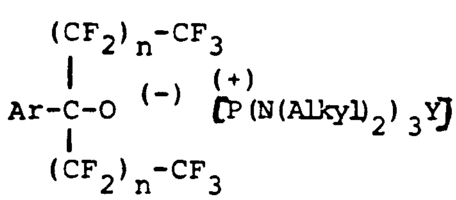
(IX)

## Patentansprüche

**1.** Ester der Formel IV

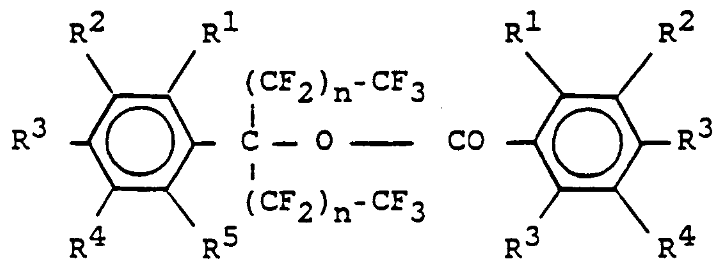 (IV),

worin die Reste $R^1$ bis $R^5$ gleich oder verschieden sind und Wasserstoff, Alkyl mit 1 bis 6 Kohlenstoffatomen, das perfluoriert sein kann, Halogen, Alkoxy oder Alkylthio mit jeweils 1 bis 6 Kohlenstoffatomen sind und n in beiden Fällen gleich oder verschieden ist und 0 oder eine ganze Zahl von 1 bis 5 bedeutet mit der Maßgabe, daß alle $R^1$ bis $R^5$ eine andere Bedeutung als jeweils Wasserstoffatome oder Fluoratome haben, wenn $n = 0$ ist, und wobei 1,1,1,3,3,3-Hexafluor-2-phenyl-propyl-2-(4-chlorbenzoat) ausgenommen ist.

**2.** Ester nach Anspruch 1, dadurch gekennzeichnet, daß an den aromatischen Ring nicht mehr als drei und vorzugsweise nur höchstens zwei Substituenten $R^1$ bis $R^5$ mit anderer Bedeutung als Wasserstoff gebunden sind.

**3.** Ester nach Anspruch 1 oder 2, dadurch gekennzeichnet, daß die Alkyl-, Alkyloxy- und Alkylthiosubstituenten zusammen höchstens 4 C-Atome enthalten.

**4.** Ester nach Anspruch 1, dadurch gekennzeichnet, daß die Reste $R^1$ bis $R^5$ in beiden aromatischen Ringen jeweils die gleiche Bedeutung haben.

**5.** Verfahren zur Herstellung von Arylbisperfluoralkylverbindungen der allgemeinen Formel VIII

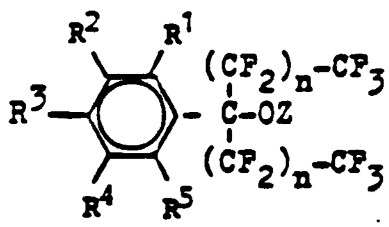 (VIII),

in der $R^1$ bis $R^5$ gleich oder verschieden sind und Wasserstoff, Alkyl mit 1 bis 6 Kohlenstoffatomen, das perfluoriert sein kann, Halogen, Alkoxy oder Alkylthio mit jeweils 1 bis 6 Kohlenstoffatomen sind und n in beiden Fällen gleich oder verschieden ist und 0 oder eine ganze Zahl von 1 bis 5 bedeutet und Z Wasserstoff oder den Arylcarbonsäurerest V

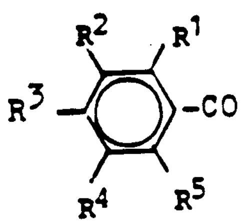 (V)

bedeutet, dadurch gekennzeichnet, daß man Carbonylverbindungen der allgemeinen Formel Va

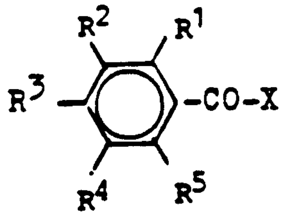

(Va)

in der $R^1$ bis $R^5$ die angegebenen Bedeutungen haben und X Fluor, Chlor, Brom oder eine Perfluoralkylgruppe $-(CF_2)_nCF_3$ ist, mit Perfluoralkylhalogeniden der allgemeinen Formel $CF_3-(CF_2)_n-Y$ (VI), worin Y Chlor, Brom oder Jod ist und wobei n Jeweils die angegebene Bedeutung hat, in Gegenwart von einer - bezogen auf die Verbindung Va-mindestens äquimolaren Menge an Phosphorigsäuretrisdialkylamiden der allgemeinen Formel $P(N(Alkyl)_2)_3$ (VII) umsetzt.

**6.** Verfahren nach Anspruch 5, dadurch gekennzeichnet, daß man eine Verbindung Va, in der X Fluor, Chlor oder Brom ist, mit einer äquimolaren Menge Perfluoralkylhalogenid VI und Phosphorigsäuretrisdialkylamid VII zu einer Verbindung VIII umsetzt, in der Z den Arylcarbonsäurerest V darstellt.

**7.** Verfahren nach Anspruch 5, dadurch gekennzeichnet, daß man eine Verbindung Va, in der X Fluor, Chlor oder Brom ist, mit einer äquimolaren Menge Perfluoralkylhalogenid VI und Phosphorigsäuretrisdialkylamid VII zu einer Verbindung VIII umsetzt, in der Z den Arylcarbonsäurerest V darstellt und diese durch alkalische Hydrolyse zu einer Verbindung VIII spaltet, in der Z Wasserstoff darstellt.

**8.** Verfahren nach Anspruch 5, dadurch gekennzeichnet, daß man eine Verbindung Va, in der X Fluor, Chlor oder Brom ist, mit der doppelt molaren Menge Perfluoralkylhalogenid VI und Phosphorigsäuretrisdialkglamid VII umsetzt und das entstandene Alkoholat nach Zugabe einer Säure zu einer Verbindung VIII spaltet, in der Z Wasserstoff darstellt.

**9.** Verfahren nach Anspruch 5, dadurch gekennzeichnet, daß man eine Verbindung Va, in der X Perfluoralkyl ist, mit einer äquimolaren Menge Perfluoralkylhalogenid VI und Phosphorigsäuretrisdialkylamid VII umsetzt und das entstandene Alkoholat nach Zugabe einer Säure zu einer Verbindung VIII spaltet, in der Z Wasserstoff darstellt, wobei der Perfluoralkylrest X gleich oder verschieden von dem Perfluoralkylrest im Perfluoralkylhalogenid VI ist.

**10.** Verfahren nach einem oder mehreren der Ansprüche 5 bis 9, dadurch gekennzeichnet, daß die Umsetzung unter wasserfreien Bedingungen in Gegenwart eines gegenüber den Reaktionsteilnehmern inerten Lösungs- oder Verdünnungsmittels, vorteilhaft einer aprotischen Flüssigkeit, durchgeführt wird.

**11.** Verfahren nach einem oder mehreren der Ansprüche 5 bis 10, dadurch gekennzeichnet, daß man Lösungs- bzw. Verdünnungsmittel und zwei Komponenten vorlegt und die dritte zudosiert, vorzugsweise in der Weise, daß man für die Herstellung der Ester der Formel VIII, worin also Z den Arylcarbonsäurerest V darstellt, das Phosphorigsäuretriamid und das Perfluoralkylhalogenid vorlegt und das Carbonsäurehalogenid zugibt bzw. für die Herstellung der tertiären Alkohole der Formel VIII, worin Z also Wasserstoff bedeutet, Carbonsäurehalogenid und Perfluoralkylhalogenid vorlegt und das Phosphorigsäuretriamid zugibt.

**12.** Verfahren nach einem oder mehreren der Ansprüche 5 bis 11, gekennzeichnet durch wenigstens eines der Merkmale, daß

a) X in Formel Va Fluor oder Chlor ist,

b) das Perfluoralkylhalogenid VI 1 bis 3 C-Atome hat, vorzugsweise aber $CF_3Br$ oder ein Perfluoralkyljodid ist,

c) im Phosphorigsäuretrisdialkylamid VII das Alkyl 1 bis 4 C-Atome hat, vorzugsweise aber Äthyl ist,

d) die Umsetzung bei Temperaturen von -100 bis +40°C durchgeführt wird, wobei bei der Umsetzung eines Perfluoralkylhalogenids mit mindestens 2 C-Atomen zweckmäßig bei einer Temperatur oberhalb -40° und vorzugsweise bis +20°C gearbeitet wird,

e) die Umsetzung ohne Anwendung von Überdruck erfolgt oder Perfluormethylhalogenide bei

höherem als Atmosphärendruck umgesetzt werden,

f) Ester der Formel VIII, worin Z also den Arylcarbonsäurerest V bedeutet, von den anderen Produkten durch Extraktion mittels eines unpolaren Lösungsmittels abgetrennt werden,

g) Alkohole der Formel VIII, in der Z also Wasserstoff bedeutet, abgetrennt werden, indem das Reaktionsgemisch unter vermindertem Druck vom Lösungsmittel befreit und der Rückstand mit Wasser behandelt wird und

h) die anderen Reaktionsteilnehmer in einer zur Carbonylverbindung Va mindestens stöchiometrischen Menge und höchstens in einem Überschuß bis zu 20 % eingesetzt werden.

## Claims

**1.** An ester of the formula IV

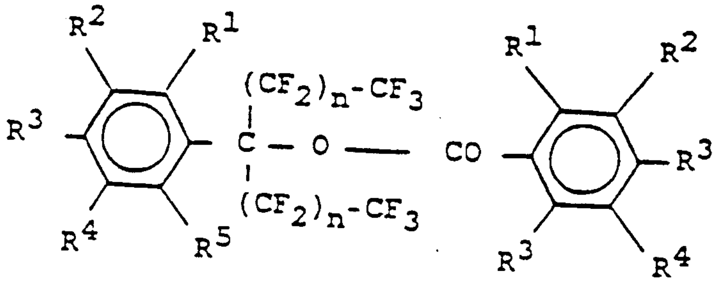

(IV)

in which the radicals $R^1$ to $R^5$ are identical or different and are hydrogen, alkyl having 1 to 6 carbon atoms which can be perfluorinated, halogen, alkoxy or alkylthio each having 1 to 6 carbon atoms and n is identical or different in both cases and is 0 or an integer from 1 to 5 with the proviso that each of $R^1$ to $R^5$ has another meaning than hydrogen atoms or fluorine atoms in each case when n = 0, and where 1,1,1,3,3,3-hexafluoro-2-phenylpropyl 2-(4-chlorobenzoate) is excluded.

**2.** An ester as claimed in claim 1, wherein no more than three and preferably only at most two substituents $R^1$ to $R^5$ having a meaning other than hydrogen are bonded to the aromatic ring.

**3.** An ester as claimed in claim 1 or 2, wherein the alkyl, alkyloxy and alkylthio substituents together contain at most 4 carbon atoms.

**4.** An ester as claimed in claim 1, wherein the radicals $R^1$ to $R^5$ in each case have the same meaning in both aromatic rings.

**5.** A process for the preparation of arylbisperfluoroalkyl compounds of the formula VIII

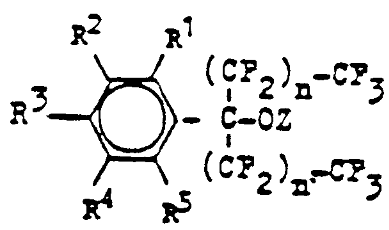

(VIII)

in which $R^1$ to $R^5$ are identical or different and are hydrogen, alkyl having 1 to 6 carbon atoms, which can be perfluorinated, halogen, alkoxy or alkylthio each having 1 to 6 carbon atoms and n is identical or different in both cases and is 0 or an integer from 1 to 5 and Z is hydrogen or the arylcarboxylic acid radical V

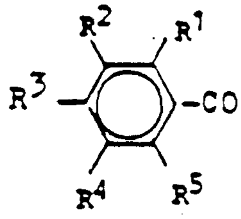

(V)

which comprises reacting carbonyl compounds of the formula Va

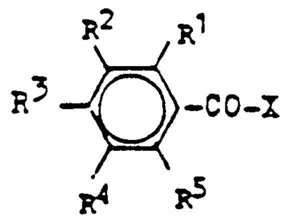

(Va)

in which $R^1$ to $R^5$ have the meanings indicated and X is fluorine, chlorine, bromine or a perfluoroalkyl group $-(CF_2)_nCF_3$, with perfluoroalkyl halides of the formula $CF_3-(CF_2)_n-Y$ (VI) in which Y is chlorine, bromine or iodine and where n in each case has the meaning indicated, in the presence of an at least equimolar amount - relative to the compound Va - of phosphorous acid trisdialkylamides of the formula $P(N(alkyl)_2)_3$ (VII).

**6.** The process as claimed in claim 5, wherein a compound Va, in which X is fluorine, chlorine or bromine, is reacted with an equimolar amount of a perfluoroalkyl halide VI and phosphorous acid trisdialkylamide VII to give a compound VIII in which Z is the arylcarboxylic acid radical V.

**7.** The process as claimed in claim 5, wherein a compound Va, in which X is fluorine, chlorine or bromine, is reacted with an equimolar amount of perfluoroalkyl halide VI and phosphorous trisdialkylamide VII to give a compound VIII in which Z is the arylcarboxylic acid radical V and this compound is cleaved by alkaline hydrolysis to give a compound VIII in which Z is hydrogen.

**8.** The process as claimed in claim 5, wherein a compound Va in which X is fluorine, chlorine or bromine is reacted with twice the molar amount of perfluoroalkyl halide VI and phosphorous acid trisdialkylamide VII and the resulting alcoholate is cleaved after addition of an acid to give a compound VIII in which Z is hydrogen.

**9.** The process as claimed in claim 5, wherein a compound Va in which X is perfluoroalkyl is reacted with an equimolar amount of perfluoroalkyl halide VI and phosphorous acid trisdialkylamide VII and the resulting alcoholate is cleaved after addition of an acid to give a compound VIII in which Z is hydrogen, where the perfluoroalkyl radical X is identical to or different from the perfluoroalkyl radical in the perfluoroalkyl halide VI.

**10.** The process as claimed in one or more of claims 5 to 9, wherein the reaction is carried out under anhydrous conditions in the presence of a solvent or diluent which is inert towards the reactants, advantageously an aprotic liquid.

**11.** The process as claimed in one or more of claims 5 to 10, wherein the solvent or diluent and two components are initially introduced and the third is metered in, preferably in the way in which, for the preparation of the esters of the formula VIII in which Z is thus the arylcarboxylic acid radical V, the phosphorous triamide and the perfluoroalkyl halide are initially introduced and the carboxylic acid halide is added or for the preparation of the tertiary alcohols of the formula VIII in which Z is thus hydrogen, carboxylic acid halide and perfluoroalkyl halide are initially introduced and the phosphorous triamide is

added.

12. The process as claimed in one or more of claims 5 to 11, which comprises at least one of the features;
a) X in formula Va is fluorine or chlorine,
b) the perfluoroalkyl halide VI has 1 to 3 carbon atoms, but is preferably $CF_3Br$ or a perfluoroalkyl iodide,
c) in the phosphorous trisdialkylamide VII the alkyl has 1 to 4 carbon atoms, but is preferably ethyl,
d) the reaction is carried out at temperatures from -100 to +40 °C, the reaction of a perfluoroalkyl halide having at least two carbon atoms expediently being carried out at a temperature above -40° and preferably up to +20°,
e) the reaction is carried out without the use of over-pressure or perfluoromethyl halides are reacted at pressures higher than atmospheric pressure,
f) esters of the formula VIII, in which Z is thus the arylcarboxylic acid radical V, are separated from the other products by extraction by means of a non-polar solvent,
g) alcohols of the formula VIII, in which Z is thus hydrogen, are separated by freeing the reaction mixture from solvent under reduced pressure and treating the residue with water and
h) the other reactants are employed in an at least stoichiometric amount relative to the carbonyl compound Va and at most in an excess of up to 20 %.

## Revendications

1. Esters répondant à la formule IV

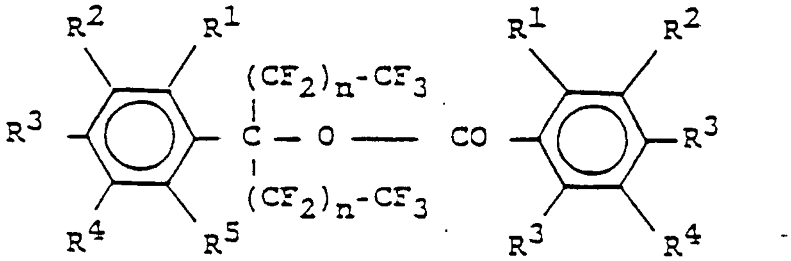

(IV)

dans laquelle $R^1$ à $R^5$ représentent chacun, indépendamment les uns des autres, l'hydrogène, un alkyle contenant de 1 à 6 atomes de carbone, qui peut être perfluoré, un halogène, un alcoxy ou un alkylthio contenant chacun de 1 à 6 atomes de carbone, et les deux indices n sont égaux ou différents et représentent chacun un entier de 0 à 5, avec la condition que les symboles $R^1$ à $R^5$ ne représentent pas tous en même temps chacun un atome d'hydrogène ni tous en même temps chacun un atome de fluor dans le cas où n est égal à zéro et que le 4-chlorobenzoate de 1,1,1,3,3,3-hexafluoro-2-phényl-propyle-(2) soit exclu.

2. Esters selon la revendication 1 caractérisés en ce que le cycle aromatique porte au plus trois et de préférence au plus deux substituants $R^1$ à $R^5$ autres que l'hydrogène.

3. Esters selon l'une des revendications 1 et 2 caractérisés en ce que les substituants alkyles, alcoxy et alkylthio contiennent ensemble au plus quatre atomes de carbone.

4. Esters selon la revendication 1 caractérisé en ce que les symboles $R^1$ à $R^5$, sur les deux cycles aromatiques, ont chacun la même signification.

5. Procédé pour préparer des composés aryl-bisperfluoralkyliques répondant à la formule générale VIII :

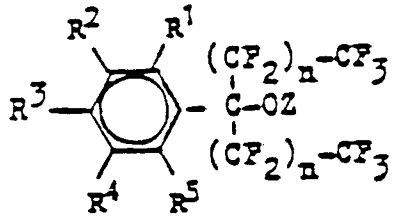

(VIII)

dans laquelle $R^1$ à $R^5$ représentent chacun, indépendamment les uns des autres, l'hydrogène, un alkyle contenant de 1 à 6 atomes de carbone, qui peut être perfluoré, un halogène, un alcoxy ou un alkylthio contenant chacun de 1 à 6 atomes de carbone, les indices n sont égaux ou différents et représentent chacun un entier de 0 à 5, et Z représente l'hydrogène ou le radical d'acide arylcarboxylique V :

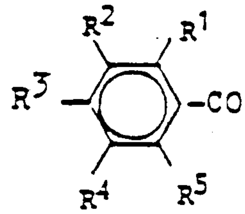

(V) .

procédé caractérisé en ce qu'on fait réagir des composés carbonyliques répondant à la formule générale Va :

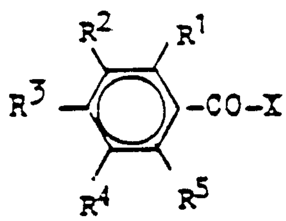

(Va)

dans laquelle $R^1$ à $R^5$ ont les significations qui leur ont été données ci-dessus et X représente le fluor, le chlore, le brome ou un radical perfluoralkyle $-(CF_2)_nCF_3$,
avec des halogénures de perfluoralkyles répondant à la formule générale VI :

$CF_3-(CF_2)_n-Y$ (VI)

dans laquelle Y représente le chlore, le brome ou l'iode, les indices n ayant à chaque fois les significations indiquées plus haut,
en présence d'une quantité au moins équimolaire -par rapport au composé Va - de tris-(dialkylamides) de l'acide phosphoreux de formule générale $P(N(alkyl)_2)_3$ (VII).

**6.** Procédé selon la revendication 5 caractérisé en ce qu'on fait réagir un composé de formule Va dans lequel X représente le fluor, le chlore ou le brome, avec une quantité équimolaire d'un halogénure de perfluoralkyle VI et d'un tris-(dialkylamide) de l'acide phosphoreux VII de manière à obtenir un composé VIII dans lequel Z représente un radical d'acide arylcarboxylique V.

**7.** Procédé selon la revendication 5 caractérisé en ce qu'on fait réagir un composé de formule Va dans lequel X représente le fluor, le chlore ou le brome, avec une quantité équimolaire d'un halogénure de perfluoralkyle VI et d'un tris-(dialkylamide) de l'acide phosphoreux VII de manière à obtenir un composé VIII dans lequel Z représente un radical d'acide arylcarboxylique V et on coupe celuici, par

hydrolyse alcaline, pour obtenir un composé VIII dans lequel Z représente l'hydrogène.

**8.** Procédé selon la revendication 5 caractérisé en ce qu'on fait réagir un composé de formule Va dans lequel X représente le fluor, le chlore ou le brome, avec une quantité double de la quantité molaire d'un halogénure de perfluoralkyle VI et d'un tris-(dialkylamide) de l'acide phosphoreux VII et on coupe l'alcoolate formé, après addition d'un acide, de manière à obtenir un composé VIII dans lequel Z représente l'hydrogène.

**9.** Procédé selon la revendication 5 caractérisé en ce qu'on fait réagir un composé de formule Va dans lequel X représente un radical perfluoralkyle, avec une quantité équimolaire d'un halogénure de perfluoralkyle VI et d'un tris-(dialkylamide) de l'acide phosphoreux VII et on coupe l'alcoolate formé, après addition d'un acide, de manière à obtenir un coposé VIII dans lequel Z représente l'hydrogène, le radical perfluoralkyle X étant identique ou différent du radical perfluoralkyle contenu dans l'halogénure de perfluoralkyle VI.

**10.** Procédé selon une ou plusieurs des revendications 5 à 9, caractérisé en ce qu'on effectue la réaction dans des conditions anhydres en présence d'un solvant ou diluant inerte à l'égard des corps participant à la réaction, avantageusement en présence d'un liquide aprotique.

**11.** Procédé selon une ou plusieurs des revendications 5 à 10, caractérisé en ce qu'on place dès le départ dans le récipient réactionnel le solvant ou diluant et deux des composantes et on introduit progressivement la troisième, de préférence en opérant de la façon suivante : pour préparer les esters de formule VIII, dans lesquels par conséquent Z représente le radical d'acide arylcarboxylique V, on place dès le départ dans le récipient le triamide de l'acide phosphoreux et l'halogénure de perfluoralkyle et on ajoute l'halogénure d'acide carboxylique, et, pour préparer des alcools tertiaires de formule VIII, dans lesquels par conséquent Z représente l'hydrogène, on place dès le départ dans le récipient l'halogénure d'acide carboxylique et l'halogénure de perfluoralkyle et on ajoute le triamide de l'acide phosphoreux.

**12.** Procédé selon une ou plusieurs des revendications 5 à 11, caractérisé par une ou plusieurs des particularités suivantes :

a) le symbole X, dans la formule Va, représente le fluor ou le chlore,

b) l'halogénure de perfluoralkyle VI contient de 1 à 3 atomes de carbone mais est de préférence $CF_3Br$ ou un iodure de perfluoralkyle,

c) dans le tris-(dialkylamide) de l'acide phosphoreux VII l'alkyle contient de 1 à 4 atomes de carbone mais est de préférence un radical éthyle,

d) la réaction est effectuée à des températures de -100 à +40 °C, et, dans le cas où l'on fait réagir un halogénure de perfluoralkyle contenant au moins 2 atomes de carbone, elle est avantageusement exécutée à une température supérieure à -40 ° et allant de préférence jusqu'à +20 °C,

e) on effectue la réaction sans surpression ou on fait réagir des halogénures de perfluorométhyle sous une pression supérieure à la pression atmosphérique,

f) on sépare les esters de formule VIII, dans lesquels par conséquent Z représente le radical d'acide arylcarboxylique V, des autres produits par extraction au moyen d'un solvant non polaire,

g) on sépare les alcools de formule VIII, dans lesquels par conséquent Z représente l'hydrogène, en chassant le solvant du mélange réactionnel sous pression réduite et en traitant le résidu par de l'eau et

h) les autres partenaires réactionnels sont mis en jeu en une quantité au moins égale à la quantité stoechiométrique par rapport au composé carbonylique Va et au plus en un excès de 20 %.